# EUROPÄISCHE PATENTSCHRIFT

(11) **EP 3 777 228 B1**
(45) Veröffentlichungstag und Bekanntmachung des Hinweises auf die Patenterteilung: **16.08.2023**
(21) Anmeldenummer: 19715033.7
(22) Anmeldetag: 27.03.2019
(51) Int. Cl.: H04R 1/10, A61F 11/14

(54) **GEHÖRSCHUTZ MIT BRILLE**
HEARING PROTECTOR WITH SPECTACLES
PROTECTION AUDITIVE AVEC LUNETTES

(30) Priorität: 04.04.2018 DE 102018107957
(43) Veröffentlichungstag der Anmeldung: 17.02.2021
(73) Patentinhaber: Pfanner Schutzbekleidung GmbH, 6842 Koblach (AT)
(72) Erfinder: PFANNER, Anton, 6845 Hohenems (AT)
(74) Vertreter: Schumacher & Willsau
(86) Internationale Anmeldenummer: PCT/EP2019/057725
(87) Internationale Veröffentlichungsnummer: WO 2019/192906

(56) Entgegenhaltungen:
- EP-A2- 0 818 186
- WO-A1-2011/038486
- US-A- 5 289 592
- US-A1- 2013 047 322
- US-A1- 2016 015 566
- US-B1- 9 235 062

## Beschreibung

Die Erfindung betrifft einen Gehörschutz mit Brille, wobei der Gehörschutz mindestens einen Ohrschützer und einen Kopfbügel aufweist und wobei die Brille zwei Brillenbügel zur Befestigung der Brille an dem Gehörschutz aufweist.

Ein solcher Gehörschutz wird insbesondere in Umgebungen eingesetzt, in denen das Gehör und die Augen beziehungsweise das Gesicht von Personen geschützt werden sollen. Hierdurch wird zum Beispiel eine erhöhte Sicherheit am Arbeitsplatz hergestellt, beispielsweise bei Forstarbeitern, auf Baustellen oder bei sonstigen Beschäftigungen, die mit einer Gefährdung des Gehörs und der Augen von Personen einhergehen. Ebenfalls werden Brillen mit getönten Scheiben als Sonnenschutz eingesetzt (siehe US2013/047322 A1).

Vorliegend wird der Begriff "Gehörschutz" verwendet. Dieser ist sehr allgemein zu verstehen. Er bezieht sich auch auf Vorrichtungen, die zur Kommunikation oder auch nur zum Hören von Musik beziehungsweise zur Wahrnehmung sonstiger Kommunikationen vorgesehen sind. Auch ein herkömmlicher Kopfhörer mit Ohrmuscheln und Kopfbügeln ist insofern ein "Gehörschutz" im Sinne der vorliegenden Offenbarung. Das gattungsgemäße Zusammenrücken von "Gehörschutz" und "Kopfhörer" wird dadurch augenscheinlich, als moderne Gehörschutzeinrichtungen vielfach mit Kommunikationsmitteln ausgestattet sind. Mit anderen Worten, die schützenden Ohrschützer enthalten vielfach einen Lautsprecher und möglicherweise eine Schnittstelle für ein Mikrofon, um den betroffenen Personen eine Kommunikation zu ermöglichen.

Ein gattungsgemäßer Gehörschutz ist aus der US 4,802,243 bekannt. An dem Kopfbügel des Gehörschutzes sind Tragbügel angebracht. Diese tragen die Ohrschützer. Eine Brille ist ebenfalls an den Tragbügeln schwenkbar befestigt. Dabei handelt es sich um eine vergleichsweise aufwendige Konstruktion.

Der Erfindung liegt die Aufgabe zugrunde, einen gattungsgemäßen Gehörschutz so weiterzubilden, dass eine möglichst einfache Konstruktion vorliegt. Ferner soll erreicht werden, dass der Gehörschutz variabel ausgestattet werden kann.

Diese Aufgabe wird mit den Merkmalen des unabhängigen Anspruchs gelöst. Vorteilhafte Ausführungsformen der Erfindung sind in den abhängigen Ansprüchen angegeben.

Die Erfindung baut auf dem gattungsgemäßen Gehörschutz dadurch auf, dass die Befestigung der Brille an dem Gehörschutz durch Befestigung der Brillenbügel an dem Kopfbügel erfolgt. Die Befestigung der Brille am Kopfbügel des Gehörschutzes ist die Grundlage für eine besonders einfache konstruktive Lösung. Der Befestigungspunkt der Brille an dem Gehörschutz ist vorzugsweise so weit von einem Ohrschützer, der mit dem Kopfbügel verbunden werden kann, entfernt, dass die Brille und der Ohrschützer unabhängig bewegt werden können und sich insbesondere nicht in ihrer Bewegung behindern. Es ist nicht erforderlich, den Gehörschutz mit Tragbügeln für die Ohrschützer auszustatten, welche selber die Möglichkeit bieten, die Brille daran zu befestigen. Vielmehr kann der Gehörschutz mit verschiedenartigen Tragbügeln ausgestattet werden, denn die Schnittstelle zwischen Brille und Gehörschutz ist dem konstruktiven Basiselement des Gehörschutzes, nämlich dem Kopfbügel, zugeordnet. Somit ist es letztlich der konstruktive Aufbau von Kopfbügel und Brille, in welchem der Grundgedanke der Erfindung verankert ist. Hiervon ausgehend, weist der Gehörschutz eine hohe konstruktive Variabilität auf. Es ist zu bemerken, dass neben den eingangs erwähnten unterschiedlichen Arten eines Gehörschutzes, nämlich ohne Kommunikationsmittel, mit Kommunikationsmittel oder als bloßer Kopfhörer, auch unterschiedliche Brillen zum Einsatz kommen können. So kann die Brille eine rein mechanische Schutzfunktion aufweisen, sie kann am Rand offen sein, um eine gute Hinterlüftung zu ermöglichen. Sie kann am Rand geschlossen sein, um auch ein Eindringen irgendwelcher schädlicher Substanzen vom Rand her zu unterbinden, beispielsweise von Chemikalien. Die Brille kann einen Sonnenschutz zur Verfügung stellen. Ebenfalls kann die Brille eine Sehhilfe sein, also sogenannte optische Gläser aufweisen. Die Brille kann auch so ausgebildet sein, dass sie sich auch als Visier bezeichnen lässt. Dabei kann es sich um ein Vollvisier oder um ein Halbvisier handeln. Alle vorstehend genannten Eigenschaften einer Brille sind teilweise oder insgesamt kombinierbar.

Nützlicherweise ist vorgesehen, dass der Kopfbügel mindestens eine Schwenkachse aufweist, an der der mindestens eine Ohrschützer schwenkbar gelagert ist, und dass ein Brillenbügel an der mindestens einen Schwenkachse befestigt ist. Die für den Ohrschützer vorgesehene Schwenkachse dient dazu, dass der Gehörschutz bequem aufgesetzt werden kann, und sie stellt eine variable und damit sichere Anlage des Gehörschutzes am Gehör des Trägers zur Verfügung. Die Schwenkachse erhält auf der Grundlage dieser Ausführungsform eine Doppelfunktion. Sie dient nicht nur zur Befestigung der Ohrschützer, sondern auch zur Befestigung der Brille. Hierdurch wird eine besonders einfache und rationelle Konstruktion zur Verfügung gestellt.

Weiterhin ist es von besonderem Vorteil, dass die mindestens eine Schwenkachse Aufnahmen an ihren Enden aufweist und dass das der Brillenbügel über in die Aufnahmen passende Gegenstücke an der mindestens einen Schwenkachse befestigt ist. Die an der Brille vorgesehenen Gegenstücke passen in die Aufnahmen der Schwenkachse, so dass der Brille hierdurch sicherer Halt am Kopfbügel gewährt wird. Die Gegenstücke sind an klammerartigen Endstücken der Brillenbügel angeordnet, wobei die klammerartigen Endstücke den Kopfbügel formschlüssig und/oder kraftschlüssig umgreifen.

Dabei ist es von Vorteil, dass die Gegenstücke Zapfen sind.

Die Erfindung ist in besonders nützlicher Weise dadurch weitergebildet, dass mindestens ein Brillenbügel mindestens zweiteilig ist, wobei ein Kopfbügelteil gelenkig mit einem Bügelvorderteil gekoppelt ist. Durch die Mehrteiligkeit der Brillenbügel bleibt die Brille einerseits fest an dem Kopfbügel befestigt. Gleichwohl ist die Brille beweglich, so dass sie zum Beispiel aus dem Gesichtsfeld des Trägers herausgeklappt werden kann, beispielsweise nach oben. Dabei sind die Abmessungen der miteinander in Verbindung stehenden Teile der Brillenbügel so gewählt, dass die Brille in einer oberen Position verharren kann, ohne durch ihr eigenes Gewicht wieder herabzufallen. die gelenkige Konstruktion der Brillenbügel verbessert auch den Sitz der Brille, denn die Gelenke lassen es zu, dass die Brille auch bei unterschiedlichen Gesichtsformen stets ihren sicheren Sitz findet.

Besonders nützlich ist es, dass mindestens ein Brillenbügel mindestens dreiteilig ist, wobei ein Kopfbügelteil gelenkig mit einem Zwischenteil gekoppelt ist und ein Bügelvorderteil in dem Zwischenteil verschiebbar gelagert ist. Hierdurch ist die Brille nicht nur schwenkbar an dem Kopfbügel befestigt, sondern die Brillenbügel sind auch noch in ihrer Länge verstellbar. Hierdurch kann der Sitz der Brille variiert werden, beispielsweise um unter verschiedenen Arbeitsbedingungen unterschiedliche Einstellungen zu wählen. Ferner wird so sichergestellt, dass der Gehörschutz jedem Träger passt. Die Gelenkigkeit kann auch zwischen Bügelvorderteil und Zwischenteil vorliegen, während dann die Verschiebbarkeit zwischen Kopfbügelteil und Zwischenteil angeordnet ist.

In diesem Zusammenhang ist es von Vorteil, dass das Bügelvorderteil in dem Zwischenteil mindestens zwei Rastpositionen einnehmen kann. Die Rastpositionen stellen sicher, dass sich die Brillenbügel nicht unabsichtlich in ihrer Länge verändern.

Weiterhin kann der erfindungsgemäße Gehörschutz derart ausgeführt sein, dass der mindestens eine Ohrschützer über einen gabelartig ausgebildeten Tragbügel an der mindestens einen Schwenkachse gelagert ist. Die gabelartig ausgebildeten Tragbügel sind über die Schwenkachse, an der auch die Brille befestigt ist, schwenkbar mit dem Kopfbügel verbunden. Die eigentlichen Ohrschützer können ohne weiteres in die Tragbügel eingehängt werden.

Dabei ist es besonders nützlich, dass der Ohrschützer über zwei Zapfen an dem Tragbügel gelagert ist. Die Lagerung des Ohrschützers über Zapfen an dem Tragbügel ermöglicht, dass der Ohrschützer im Tragbügel schwenkbar ist.

Die Erfindung betrifft weiterhin eine Brille zur Verwendung mit einem erfindungsgemäßen Gehörschutz.

Weiterhin betrifft die Erfindung einen Kopfbügel zur Verwendung mit einem erfindungsgemäßen Gehörschutz.

Die Erfindung nun mit Bezug auf die begleitenden Zeichnungen anhand besonders bevorzugter Ausführungsformen beispielhaft erläutert.
Figur 1 zeigt eine Frontalansicht eines erfindungsgemäßen Gehörschutzes.
Figur 2 zeigt eine perspektivische Seitenansicht eines erfindungsgemäßen Gehörschutzes.
Figur 3 zeigt eine perspektivische Seitenansicht eines erfindungsgemäßen Gehörschutzes, wobei die Brille einseitig von dem Kopfbügel gelöst ist.
Figur 4 zeigt eine Einzelheit der Befestigungseinrichtung, mit der ein Brillenbügel an dem Kopfbügel befestigt werden kann.
Figur 5 zeigt eine Einzelheit der Befestigungseinrichtung, an der ein Brillenbügel befestigt werden kann.
Figur 6 zeigt eine Einzelheit des Gehörschutzes, wobei die dreiteilige Konstruktion eines Brillenbügels erkennbar ist.
Figur 7 zeigt eine Person mit aufgesetztem Gehörschutz.

Bei der nachfolgenden Beschreibung der Zeichnungen bezeichnen gleiche Bezugszeichen gleiche oder vergleichbare Komponenten.

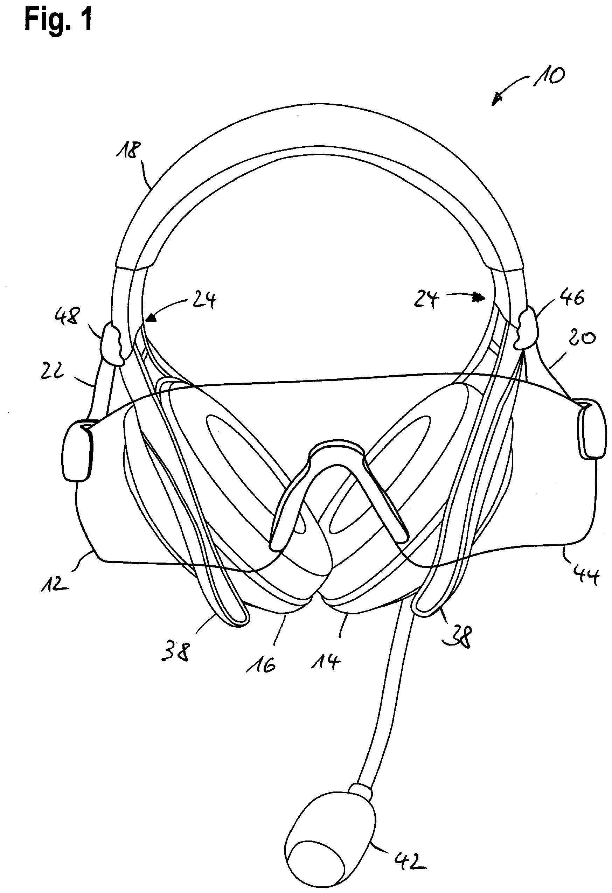

Figur 1 zeigt eine Frontalansicht eines erfindungsgemäßen Gehörschutzes 10. Der Gehörschutz 10 umfasst einen Kopfbügel 18. An dem Kopfbügel 18 sind Tragbügel 38 gelenkig angebracht. Die Schwenkachsen 24, die die Gelenkigkeit zur Verfügung stellen, verlaufen im Wesentlichen in Blickrichtung des Betrachters von Figur 1. An den Tragbügeln 38 sind Ohrschützer 14, 16 gelenkig angebracht. Die Gelenkigkeit wird durch Zapfen 40 und entsprechende Aufnahmen an den Ohrschützern 14, 16 zur Verfügung gestellt. Weder die Zapfen noch die Aufnahmen sind in der Darstellung erkennbar, da sie von den Tragbügeln 38 beziehungsweise den Ohrschützern 14, 16 verdeckt werden. Ein Ohrschützer 14 ist ein mit Kommunikationsmitteln ausgestatteter Ohrschützer. Er enthält einen Lautsprecher, und an ihm ist ein Mikrofon 42 angeschlossen. Die Kommunikationsmittel können über eine drahtlose Funkkommunikationsschnittstelle, beispielsweise eine Bluetooth-Schnittstelle arbeiten. Der andere Ohrschützer 16 ist ein passiver Ohrschützer, das heißt er dient nur zum Schutz des Ohres, und er enthält keine Kommunikationsmittel. Es ist ebenfalls möglich, dass auch der andere Ohrschützer 16 mit Kommunikationsmitteln ausgestattet ist, ähnlich dem ersten Ohrschützer 14. In einer anderen Variante ist einer der Ohrschützer, beispielsweise der erste Ohrschützer 14 mit Kommunikationsmitteln ausgestattet, wie beschrieben, während der andere Ohrschützer, zum Beispiel der Ohrschützer 16, es ermöglicht, Musik zu hören, beispielsweise über eine Digitalradiofunktion. Diese muss nicht notwendigerweise in den Ohrschützer 16 integriert sein, sondern sie kann unter Zuhilfenahme einer Digitalradiofunktion eines Mobiltelefons, insbesondere eines Smartphones, zur Verfügung gestellt werden, so dass es ausreicht, wenn in dem Ohrschützer 16 Mittel zum Empfangen, eventuell auch zum Senden, sowie Mittel zur Wiedergabe von Schall vorgesehen sind. An dem Kopfbügel 18 ist weiterhin eine Brille 12 befestigt. Diese Brille 12 umfasst neben anderen Komponenten eine transparente Scheibe 44 sowie daran angebrachte Brillenbügel 20, 22. An den Enden der Brillenbügel 20, 22 sind klammerartige Halter 46, 48 vorgesehen. Diese Halter 46, 48 umgreifen den Kopfbügel 18 teilweise.

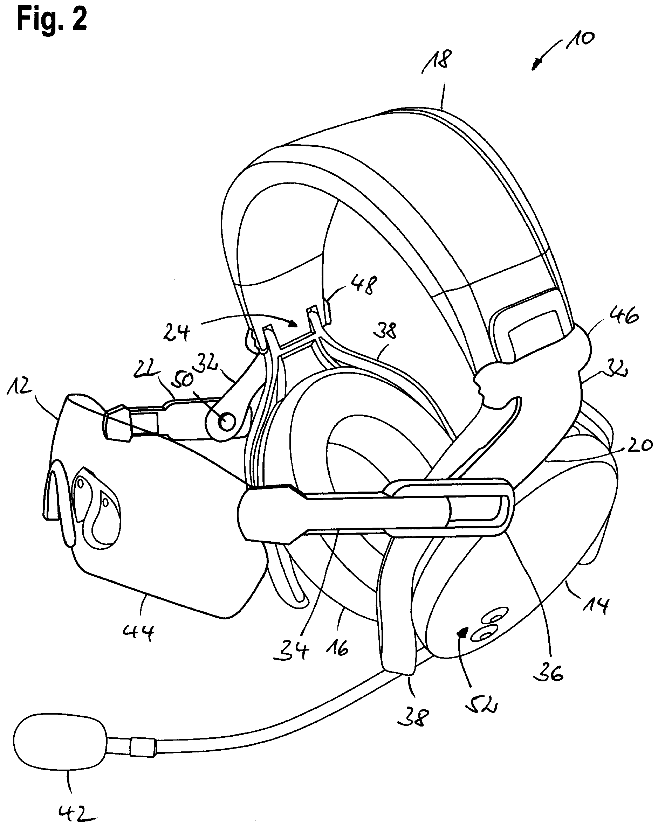

Figur 2 zeigt eine perspektivische Seitenansicht eines erfindungsgemäßen Gehörschutzes 10. Hier ist die Mehrteiligkeit der Brillenbügel 20, 22 erkennbar. Jeder Brillenbügel 20, 22 umfasst ein Kopfbügelteil 32, wobei am Ende von jedem Kopfbügelteil 32 der Halter 46, 48 vorgesehen ist, über den die Brillenbügel 20, 22 mit dem Kopfbügel 18 verbunden sind. Jedes Kopfbügelteil 32 ist mit einem Zwischenteil 36 über jeweils eine Gelenkachse 50 verbunden. Diese Gelenkachsen 50 ermöglichen es, die Brille 12 nach oben, das heißt in Richtung auf den Kopfbügel 18 zu verschwenken. Die Zwischenteile 36 nehmen Bügelvorderteile 34 verschiebbar auf. Es ist eine Zwischenstellung dargestellt. Das Bügelvorderteil 34 kann weiter aus dem Zwischenteil 36 herausgezogen werden beziehungsweise es kann weiter in das Zwischenteil 36 hineingeschoben werden. Auf diese Weise wird die Länge der Brillenbügel 20, 22 insgesamt verändert. Figur 2 zeigt ferner in gewissem Detail an welcher Stelle die Schwenkachse 24, über die die Tragbügel 38 mit dem Kopfbügel 18 verbunden sind, relativ zu den Haltern 46, 48 der Brillenbügel 20, 22 liegt. Insbesondere verbindet die Schwenkachse 24 die klammerartigen Fortsätze der Halter 46, 48. Der aktive Ohrschützer 14 hat an seiner Außenseite Bedienelemente 52 zum Steuern der Kommunikation.

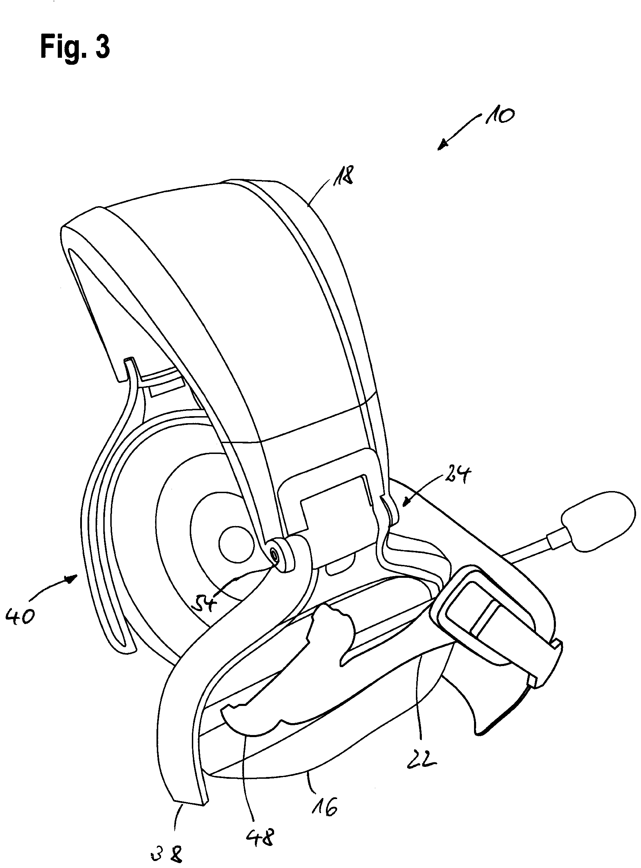

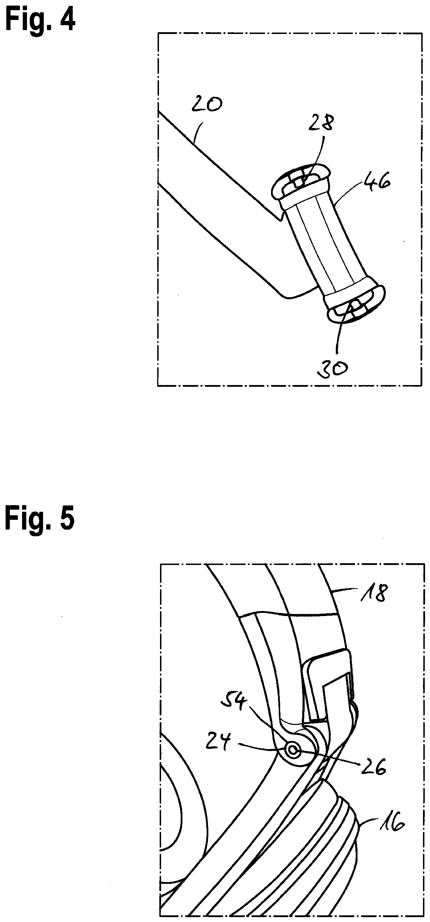

Figur 3 zeigt eine perspektivische Seitenansicht eines erfindungsgemäßen Gehörschutzes 10, wobei die Brille 12 einseitig von dem Kopfbügel 18 gelöst ist. Aufgrund des von seiner Befestigung gelösten Halters 48 erkennt man nun genauer die Schwenkachse 24, über die die Träger 38 mit dem Tragbügel 18 verbunden sind. Eine solche Schwenkachse 24 umfasst einen Stift 54, der jeweils ein Ende des Kopfbügels 18 sowie die Träger 38 durchdringt. Die Enden von jedem Stift 54 weisen Aufnahmen 26 (siehe Figur 5) auf. Wenn die Halter 46, 48 den Tragbügel 18 in diesem Bereich umklammern, werden Zapfen 28, 30 (siehe Figur 4) von den Aufnahmen 54 aufgenommen, so dass die Halter 46, 48 sicheren Halt an dem Kopfbügel 18 finden.

Figur 4 zeigt eine Einzelheit der Befestigungseinrichtung, mit der ein Brillenbügel 20, 22 an dem Kopfbügel 18 befestigt werden kann. Der Halter 46, der als ein klammerartiges Endstück eines Brillenbügels 20 ausgebildet ist, umfasst einen in Blickrichtung hinten liegendes Mitteilteil, sowie zwei Endstücke, die dem Betrachter in Blickrichtung entgegenspringen. An den Endstücken sind Zapfen 28, 30 angeordnet, welche sich im Wesentlichen in der Papierebene der Figur 4 erstrecken. Diese Zapfen 28, 30 finden Halt in den Aufnahmen 24 (siehe Figur 5).

Figur 5 zeigt eine Einzelheit der Befestigungseinrichtung, an der ein Brillenbügel 20, 22 befestigt werden kann. Hier ist der als Schwenkachse 24 dienende Stift 54 endseitig erkennbar und durch zwei konzentrische Kreise dargestellt. Der innere Kreis markiert dabei den Außenumfang einer Aufnahme 26 für einen der Zapfen 28, 30 (siehe Figur 4).

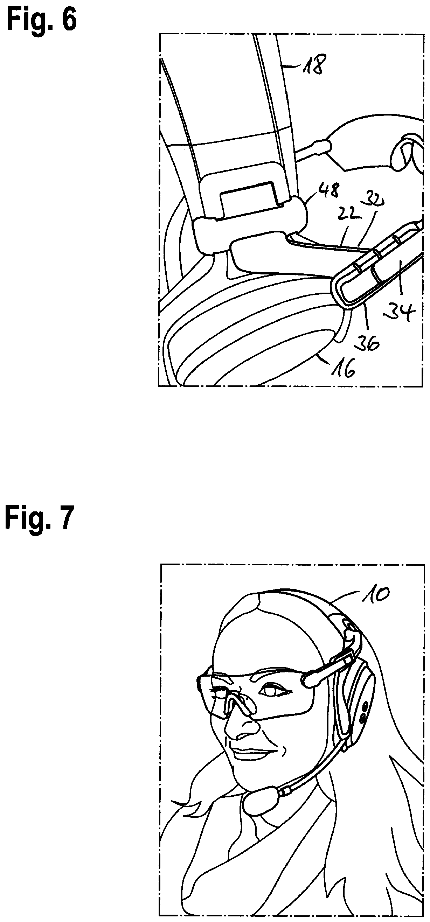

Figur 6 zeigt eine Einzelheit des Gehörschutzes 10, wobei die dreiteilige Konstruktion eines Brillenbügels 20, 22 erkennbar ist. Insofern umfasst er ein Kopfbügelteil 32 mit Halter 48, ein Zwischenteil 36 und ein Bügelvorderteil 34. Das Zwischenteil 36 hat drei Markierungen, welche Rastpositionen des Bügelvorderteils 34 in dem Zwischenteil 36 markieren.

Figur 7 zeigt eine Person mit aufgesetztem Gehörschutz 10.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

### Bezugszeichenliste

- 10: Gehörschutz
- 12: Brille
- 14: Ohrschützer
- 16: Ohrschützer
- 18: Kopfbügel
- 20: Brillenbügel
- 22: Brillenbügel
- 24: Schwenkachse
- 26: Aufnahmen
- 28: Zapfen
- 30: Zapfen
- 32: Kopfbügelteil
- 34: Bügelvorderteil
- 36: Zwischenteil
- 38: Tragbügel
- 40: Zapfen
- 42: Mikrofon
- 44: Scheibe
- 46: Halter
- 48: Halter
- 50: Gelenkachse
- 52: Bedienelement
- 54: Stift

## Patentansprüche

1. Gehörschutz (10) mit Brille (12),
- wobei der Gehörschutz (10) mindestens einen Ohrschützer (14, 16) und einen Kopfbügel (18) aufweist und
- wobei die Brille (12) zwei Brillenbügel (20, 22) zur Befestigung der Brille (12) an dem Gehörschutz (10) aufweist,
- wobei die Befestigung der Brille (12) an dem Gehörschutz (10) durch Befestigung der Brillenbügel (20, 22) an dem Kopfbügel (18) erfolgt;
**dadurch gekennzeichnet, dass** der Kopfbügel (18) mindestens eine Schwenkachse (24) aufweist, an der ein gabelartig ausgebildeter Tragbügel (38) und der mindestens eine Ohrschützer (14, 16) schwenkbar gelagert sind, und dass ein Brillenbügel (20, 22) an der mindestens einen Schwenkachse (24) befestigt ist.

2. Gehörschutz (10) mit Brille (12) nach Anspruch 1, **dadurch gekennzeichnet, dass** die mindestens eine Schwenkachse (24) Aufnahmen (26) an ihren Enden aufweist und dass der Brillenbügel (20, 22) über in die Aufnahmen (26) passende Gegenstücke an der mindestens einen Schwenkachse (24) befestigt ist.

3. Gehörschutz (10) mit Brille (12) nach Anspruch 2, **dadurch gekennzeichnet, dass** die Gegenstücke Zapfen (28, 30) sind.

4. Gehörschutz (10) mit Brille (12) nach einem der vorhergehenden Ansprüche, **dadurch gekennzeichnet, dass** mindestens ein Brillenbügel (20, 22) mindestens zweiteilig ist, wobei ein Kopfbügelteil (32) gelenkig mit einem Bügelvorderteil (34) gekoppelt ist.

5. Gehörschutz (10) mit Brille (12) nach einem der vorhergehenden Ansprüche, **dadurch gekennzeichnet, dass** mindestens ein Brillenbügel (20, 22) mindestens dreiteilig ist, wobei ein Kopfbügelteil (32) gelenkig mit einem Zwischenteil (36) gekoppelt ist und ein Bügelvorderteil (34) in dem Zwischenteil (36) verschiebbar gelagert ist.

6. Gehörschutz (10) mit Brille (12) nach Anspruch 5, **dadurch gekennzeichnet, dass** das Bügelvorderteil (34) in dem Zwischenteil (36) mindestens zwei Rastpositionen einnehmen kann.

7. Gehörschutz (10) mit Brille (12) nach einem der vorhergehenden Ansprüche, **dadurch gekennzeichnet, dass** der Ohrschützer (14, 16) über zwei Zapfen (40) an dem Tragbügel (38) gelagert ist.

## Claims

1. A hearing protector (10) comprising spectacles (12),
- the hearing protector (10) comprising at least one ear protector (14, 16) and a headband (18), and
- the spectacles (12) comprising two temples (20, 22) for fastening the spectacles (12) to the hearing protector (10),
- wherein the attachment of the spectacles (12) to the hearing protector (10) is realised by fastening the temples (20, 22) to the headband (18);
**characterised in that** the headband (18) has at least one pivoting axis (24) on which a bifurcated support bracket (38) and the at least one ear protector (14, 16) are pivotably supported, and **in that** a temple (20, 22) is attached to the at least one pivoting axis (24).

2. The hearing protector (10) comprising spectacles (12) according to claim 1, **characterised in that** the at least one pivoting axis (24) comprises receptacles (26) at its ends and **in that** the temple (20, 22) is attached to the at least one pivoting axis (24) via mating parts fitting into the receptacles (26).

3. The hearing protector (10) comprising spectacles (12) according to claim 2, **characterised in that** the mating parts are plugs (28, 30).

4. The hearing protector (10) comprising spectacles (12) according to one of the preceding claims, **characterised in that** at least one temple (20, 22) is comprised of at least two parts, wherein a headband part (32) is jointedly coupled to a temple front portion (34).

5. The hearing protector (10) comprising spectacles (12) according to one of the preceding claims, **characterised in that** at least one temple (20, 22) is comprised of at least three parts, wherein a headband part (32) is jointedly coupled to an intermediate part (36), and a temple front portion (34) is slidably supported in the intermediate part (36).

6. The hearing protector (10) comprising spectacles (12) according to claim 5, **characterised in that** the temple front portion (34) in the intermediate part (36) can assume at least two latched positions.

7. The hearing protector (10) comprising spectacles (12) according to one of the preceding claims, **characterised in that** the ear protector (14, 16) is supported on the support bracket (38) via two plugs (40).

## Revendications

1. Protection auditive (10) avec lunettes (12),
- la protection auditive (10) comprenant au moins un protège-oreille (14, 16) et un serre-tête (18), et
- les lunettes (12) présentent deux branches de lunettes (20, 22) pour la fixation des lunettes (12) sur la protection auditive (10),
- la fixation des lunettes (12) sur la protection auditive (10) s'effectuant par fixation des branches de lunettes (20, 22) au serre-tête (18) ;
**caractérisée en ce que** le serre-tête (18) présente au moins un axe de pivotement (24) sur lequel un étrier de support (38) réalisé en forme de fourche et l'au moins un protège-oreille (14, 16) sont montés de manière pivotante, et **en ce qu'**une branche de lunettes (20, 22) est fixée sur l'au moins un axe de pivotement (24).

2. Protection auditive (10) avec lunettes (12) selon la revendication 1, **caractérisée en ce que** l'au moins un axe de pivotement (24) présente des logements (26) à ses extrémités et **en ce que** la branche de lunettes (20, 22) est fixée à l'au moins un axe de pivotement (24) par des contreparties s'adaptant dans les logements (26).

3. Protection auditive (10) avec lunettes (12) selon la revendication 2, **caractérisée en ce que** les contreparties sont des tourillons (28, 30).

4. Protection auditive (10) avec lunettes (12) selon l'une quelconque des revendications précédentes, **caractérisée en ce qu'**au moins une branche de lunettes (20, 22) est au moins en deux parties, une partie de serre-tête (32) étant couplée de manière articulée à une partie avant de branche (34).

5. Protection auditive (10) avec lunettes (12) selon l'une quelconque des revendications précédentes, **caractérisée en ce qu'**au moins une branche de lunettes (20, 22) est au moins en trois parties, une partie de serre-tête (32) étant couplée de manière articulée à une partie intermédiaire (36) et une partie avant de branche (34) étant logée de manière coulissante dans la partie intermédiaire (36).

6. Protection auditive (10) avec lunettes (12) selon la revendication 5 **caractérisée en ce que** la partie avant de la branche (34) peut prendre au moins deux positions d'arrêt dans la partie intermédiaire (36).

7. Protection auditive (10) avec lunettes (12) selon l'une des revendications précédentes, **caractérisée en ce que** le protège-oreille (14, 16) est monté sur l'étrier de support (38) par l'intermédiaire de deux tourillons (40).
